(19) Europäisches Patentamt
European Patent Office
Office européen des brevets

(11) Veröffentlichungsnummer : **0 205 076**
**B1**

(12) # EUROPÄISCHE PATENTSCHRIFT

(45) Veröffentlichungstag der Patentschrift : **04.05.88**

(51) Int. Cl.⁴ : **C 07 C 147/06**, A 01 N 47/12

(21) Anmeldenummer : **86107524.0**

(22) Anmeldetag : **03.06.86**

(54) **Benzaldoxim-carbamat-Derivate.**

(30) Priorität : **12.06.85 DE 3520943**

(43) Veröffentlichungstag der Anmeldung : **17.12.86 Patentblatt 86/51**

(45) Bekanntmachung des Hinweises auf die Patenterteilung : **04.05.88 Patentblatt 88/18**

(84) Benannte Vertragsstaaten : **AT BE CH DE FR GB IT LI NL SE**

(56) Entgegenhaltungen :
**CH-A- 423 350**
**US-A- 3 721 711**
**US-A- 4 278 613**

(73) Patentinhaber : **BAYER AG**
**Konzernverwaltung RP Patentabteilung**
**D-5090 Leverkusen 1 Bayerwerk (DE)**

(72) Erfinder : **Fest, Christa, Dr.**
**Im Johannistal 20**
**D-5600 Wuppertal 1 (DE)**
Erfinder : **Brandes, Wilhelm, Dr.**
**Eichendorffstrasse 3**
**D-5653 Leichlingen (DE)**
Erfinder : **Hänssler, Gerd, Dr.**
**Am Arenzberg 58a**
**D-5090 Leverkusen 3 (DE)**
Erfinder : **Reinecke, Paul, Dr.**
**Steinstrasse 8**
**D-5090 Leverkusen 3 (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 205 076 B1

**Beschreibung**

Die vorliegende Erfindung betrifft neue Benzaldoxim-carbamat-Derivate, ein Verfahren zu ihrer Herstellung und ihre Verwendung als Schädlingsbekämpfungsmittel.

Es sind bereits eine Reihe von Benzaldoximen, wie z.B. das α-Phenylsulfonyl-2,6-dichlor-benzaldoxim, bekannt, ebenso deren Verwendung als Pflanzenschutzmittel ; vor allem ist ihre Verwendung in Mitteln zur Bekämpfung von Weizensteinbrand (vgl. Schweizer Patent Nr. 423.350) bekannt.

Weiterhin sind organische Schwefelverbindungen, wie Metallsalze der Dithiocarbamidsäuren, z.B. das Zinkethylen-1,2-bis-dithiocarbamat, als gute Fungizide bekannt (vgl. z.B. R. Wegler, « Chemie der Pflanzenschutz-und Schädlingsbekämpfungsmittel », Band 2, Seite 65, Springer-Verlag, Berlin-Heidelberg-New York, 1970).

Ihre Wirkung ist jedoch unter bestimmten Bedingungen nicht immer voll befriedigend, z.B. bei niedrigen Aufwandmengen.

Es wurden neue Benzaldoxim-carbamat-Derivate der Formel (1)

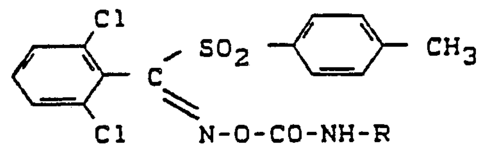

(I)

gefunden, in welcher R für Alkyl mit 1 bis 6 Kohlenstoffatomen, für Halogenalkyl mit 1 bis 8 Kohlenstoffatomen und 1 bis 5 Halogenatomen, für Cyanalkyl mit 1 bis 6 Kohlenstoffatomen im Alkylteil, für Tosyl, für Benzyl, für gegebenenfalls ein- bis fünffach durch Alkyl mit 1 bis 4 Kohlenstoffatomen substituiertes Cycloalkyl mit 5 bis 7 Kohlenstoffatomen oder für gegebenenfalls ein- bis fünffach, gleich oder verschieden durch Alkyl mit 1 bis 6 Kohlenstoffatomen, Alkoxy mit 1 bis 4 Kohlenstoffatomen, Halogenalkoxy oder Halogenalkyl mit je 1 bis 4 Kohlenstoffatomen und 1 bis 5 Halogenatomen und Halogen substituiertes Phenyl steht.

Weiterhin wurde gefunden, daß man die Benzaldoxim-carbamat-Derivate der Formel (I)

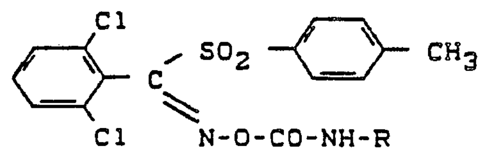

(I)

in welcher R für Alkyl mit 1 bis 6 Kohlenstoffatomen, für Halogenalkyl mit 1 bis 8 Kohlenstoffatomen und 1 bis 5 Halogenatomen, für Cyanalkyl mit 1 bis 6 Kohlenstoffatomen im Alkylteil, für Tosyl, für Benzyl, für gegebenenfalls ein- bis fünffach durch Alkyl mit 1 bis 4 Kohlenstoffatomen substituiertes Cycloalkyl mit 5 bis 7 Kohlenstoffatomen oder für gegebenenfalls ein- bis fünffach, gleich oder verschieden durch Alkyl mit 1 bis 6 Kohlenstoffatomen, Alkoxy mit 1 bis 4 Kohlenstoffatomen, Halogenalkoxy oder Halogenalkyl mit je 1 bis 4 Kohlenstoffatomen und 1 bis 5 Halogenatomen und Halogen substituiertes Phenyl steht, erhält, wenn man α-(4-Methyl-phenylsulfonyl)-2,6-dichlorbenzaldoxim der Formel (II)

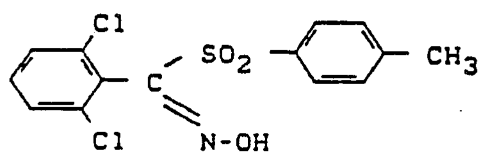

(II)

mit Isocyanaten der Formel (III)

$$R\text{—}NCO \qquad \text{(III)}$$

in welcher R die oben angegebene Bedeutung hat, gegebenenfalls in Gegenwart von Lösungs- oder Verdünnungsmitteln bei Temperaturen von 0 °C bis 100 °C umsetzt.

Die erfindungsgemäßen Benzaldoxim-carbamat-Derivate der Formel (I) weisen starke biologische, vor allem fungizide Eigenschaften auf.

Überraschenderweise zeigen dabei die erfindungsgemäßen Verbindungen eine erheblich höhere, vor allem fungizide Wirksamkeit als die aus dem Stand der Technik bekannten Verbindungen, die strukturell und/oder wirkungsmäßig sehr naheliegende Verbindungen sind.

Die erfindungsgemäßen Verbindungen der Formel (I) können als syn- oder anti-Isomere oder als deren Gemische in unterschiedlicher Zusammensetzung anfallen. Die Erfindung bezieht sich sowohl auf die reinen Isomeren als auch auf die Isomerengemische.

Die erfindungsgemäßen Benzaldoxim-carbamat-Derivate sind durch die Formel (I) allgemein definiert. Bevorzugt sind Verbindungen der Formel (I), in welcher R für geradkettiges oder verzweigtes Alkyl mit 1 bis 4 Kohlenstoffatomen, wie Methyl, Ethyl, n-Propyl, iso-Propyl, n-Butyl, iso-Butyl, sek.-Butyl und tert.-Butyl, für Halogenalkyl mit 1 bis 6 Kohlenstoffatomen und 1 bis 3 gleichen oder verschiedenen Halogenatomen, wie Fluor, Chlor, Brom und Iod, z.B. 2-Chlorethyl, 3-Chlor-n-propyl, 4-Chlor-n-butyl, 4-Chlor-n-pentyl und 6-Chlor-n-hexyl, für Cyanalkyl mit 1 bis 5 Kohlenstoffatomen, z.B. 3- oder 5-Cyan-n-pentyl, für Tosyl, für Benzyl, für gegebenenfalls ein- bis dreifach, gleich oder verschieden durch Alkyl mit 1 bis 3 Kohlenstoffatomen, wie Methyl, Ethyl, n-Propyl und iso-Propyl, substituiertes Cycloalkyl mit 6 oder 7 Kohlenstoffatomen, für gegebenenfalls ein- bis dreifach, gleich oder verschieden durch Alkyl mit 1 bis 4 Kohlenstoffatomen, wie z.B. Methyl, Ethyl, n-Propyl, iso-Propyl, n-Butyl, iso-Butyl, sek.-Butyl und tert.-Butyl, durch Alkoxy mit 1 bis 3 Kohlenstoffatomen, wie Methoxy, Ethoxy, n-Propoxy und iso-Propoxy, durch Halogenalkyl bzw. Halogenalkoxy mit 1 bis 3 Kohlenstoffatomen und 1 bis 3 Halogenatomen, wie Fluor, Chlor, Brom und Iod, wie z.B. Trifluormethyl, Trichlormethyl, Dichlorfluormethyl, 2-Chlorethyl, 2-Fluorethyl, Trifluormethoxy, Dichlorfluormethoxy, 2-Chlorethoxy und 2-Fluorethoxy und durch Halogen, wie Fluor, Chlor, Brom und Iod, substituiertes Phenyl steht.

Besonders bevorzugt sind Verbindungen der Formel (I), bei welchen R für Methyl, Ethyl, n-Propyl, iso-Propyl, n-Butyl, iso-Butyl, tert.-Butyl, 6-Chlor-n-hexyl, 5-Cyano-n-pentyl, Tosyl, Benzyl, für Cyclohexyl oder ein- bis dreifach durch Methyl oder Ethyl substituiertes Cyclohexyl, für Phenyl oder ein- bis dreifach, gleich oder verschieden durch Methyl, Ethyl, n-Propyl, iso-Propyl, Methoxy, Ethoxy, Trifluormethoxy, Trifluormethyl, Chlor und Fluor substituiertes Phenyl steht.

Insbesondere seien Verbindungen der Formel (I) genannt, bei welchen R für Methyl, Ethyl, n-Propyl, iso-Propyl, n-Butyl, iso-Butyl, tert.-Butyl, 6-Chlor-n-hexyl, 5-Cyano-n-pentyl, Benzyl, Tosyl, Cyclohexyl, 3,5,5-Trimethyl-cyclohexyl, 4-Methyl-cyclohexyl, Phenyl, 4-Trifluor-methoxy-phenyl, 2-, 3- oder 4-Methylphenyl, 3-Chlor-4-methyl-phenyl, 3-Methyl-4-chlorphenyl, 3- oder 4-Chlorphenyl, 3, 4-, 2, 6- oder 3,5-Dichlor-phenyl, 3-Trifluormethyl-phenyl, 4-Ethoxy-phenyl, 3-Chlor-4-trifluormethylphenyl oder 3,6-Di-iso-propyl-phenyl steht.

Ganz besonders die Verbindungen der Formel (I), in der R für Methyl, Ethyl, Tosyl, 4-Trifluormethoxy-phenyl oder 3,5,5-Trimethyl-cyclohexyl steht.

Verwendet man α-(4-Methyl-phenylsulfonyl)-2,6-dichlorbenzaldoxim und Methylisocyanat als Ausgangsstoffe, so läßt sich der Reaktionsablauf des erfindungsgemäßen Verfahrens durch das folgende Formelschema darstellen :

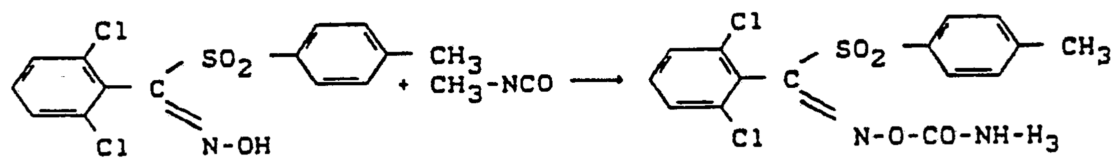

Das zur Durchführung des erfindungsgemäßen Verfahrens als Ausgangsstoff benötigte α-(4-Methyl-phenylsulfonyl)-2,6-benzaldoxim ist durch die Formel (II) definiert. Diese Verbindung und ihre Herstellung sind bekannt (vgl. z.B. Chem. Abstracts 83 : 27759e = Tetrahedron 1975, 31 (6), S. 597-600).

Die weiterhin als Ausgangsstoffe benötigten Isocyanate sind durch die Formel (III) allgemein definiert. Es handelt sich dabei um bekannte Verbindungen der organischen Chemie.

Das erfindungsgemäße Verfahren kann gegebenenfalls in Gegenwart eines Lösungs- oder Verdünnungsmittels durchgeführt werden. Als solche kommen prinzipiell alle inerten organischen Lösungsmittel in Frage. Bevorzugt verwendet man Kohlenwasserstoffe, gegebenenfalls chlorierte, wie z.B. Benzol, Toluol, Xylol, Methylenchlorid, Chloroform, Tetrachlorkohlenstoff, Chlorbenzol und Dichlorbenzol.

Die Reaktionstemperaturen können in einem größeren Bereich variiert werden. Im allgemeinen arbeitet man zwischen 0 und 100 °C, vorzugsweise zwischen 15 und 50 °C.

Bei der Durchführung des erfindungsgemäßen Verfahrens läßt man die Reaktionspartner in etwa äquimolekularen Verhältnissen Miteinander reagieren. Da die Reaktion gelegentlich exotherm ist, empfiehlt es sich zu kühlen. Die Aufarbeitung des Reaktionsgemisches erfolgt in üblicher Weise, vornehmlich durch Absaugen, Waschen und Trocknen des ausgefallenen Reaktionsproduktes.

Zur Vervollständigung der Reaktion können gegebenenfalls einige Tropfen einer Base, wie z.B. Triethylamin, zugegeben werden.

Die erfindungsgemäßen Wirkstoffe weisen eine starke mikrobizide Wirkung auf und können zur Bekämpfung von unerwünschten Mikroorganismen praktisch eingesetzt werden. Die Wirkstoffe sind für der Gebrauch als Schädlingsbekämpfungsmittel geeignet, vor allem als Fungizide.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Beispielhaft aber nich begrenzend seien einige Erreger von pilzlichen Erkrankungen, die unter die oben aufgezählten Oberbegriffe fallen, genannt :

Pythium-Arten, wie beispielsweise Pythium ultimum ;

Phytophthora-Arten, wie beispielsweise Phytophthora infestans ;

Pseudoperonospora-Arten, wie beispielsweise Pseudoperonospora humuli oder Pseudoperonospora cubense ;
Plasmopara-Arten, wie beispielsweise Plasmopara viticola ;
Peronospora-Arten, wie beispielsweise Peronospora pisi oder P. brassicae ;
Erysiphe-Arten, wie beispielsweise Erysiphe graminis ;
Sphaerotheca-Arten, wie beispielsweise Sphaerotheca fuliginea ;
Podosphaera-Arten, wie beispielsweise Podosphaera leucotricha ;
Venturia-Arten, wie beispielsweise Venturia inaequalis ;
Pyrenophora-Arten, wie beispielsweise Pyrenophora teres oder P. graminea
(Konidienform : Drechslera, Syn : Helminthosporium) ;
Cochliobolus-Arten, wie beispielsweise Cochliobolus sativus
(Konidienform : Drechslera, Syn : Helminthosporium) ;
Uromyces-Arten, wie beispielsweise Uromyces appendiculatus ;
Puccinia-Arten, wie beispielsweise Puccinia recondita ;
Tilletia-Arten, wie beispielsweise Tilletia caries ;
Ustilago-Arten, wie beispielsweise Ustilago nuda oder Ustilago avenae ;
Pellicularia-Arten, wie beispielsweise Pellicularia sasakii ;
Pyricularia-Arten, wie beispielsweise Pyricularia oryzae ;
Fusarium-Arten, wie beispielsweise Fusarium culmorum ;
Botrytis-Arten, wie beispielsweise Botrytis cinerea ;
Septoria-Arten, wie beispielsweise Septoria nodorum ;
Leptosphaeria-Arten, wie beispielsweise Leptosphaeria nodorum ;
Cercospora-Arten, wie beispielsweise Cercospora canescens ;
Alternaria-Arten, wie beispielsweise Alternaria brassicae ;
Pseudocercosporella-Arten, wie beispielseise Pseudocercosporella herpotrichoides.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens. Die erfindungsgemäßen Wirkstoffe lassen sich mit besonders gutem Erfolg zur Bekämpfung von Gemüsekrankheiten, verursacht z.B. durch Phytophthora infestans, zur Bekämpfung von Reiskrankheiten, verursacht z.B. durch Pyricularia oryzae und zur Bekämpfung von Getreidekrankheiten, verursacht z.B. durch Leptosphaeria nodorum oder Pyrenophora teres einsetzen.

Die Wirkstoffe können in die üblichen Formulierungen übergeführt werden, wie Lösungen, Emulsionen, Suspensionen, Pulver, Schaüme, Pasten, Granulate, Aerosole, Feinstverkapselungen in polymeren Stoffen und in Hüllmassen für Saatgut, sowie ULV-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage : Aromaten, wie Xylol, Toluol oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Parffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser. Mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid. Als feste Trägerstoffe kommen in Frage : z.B. natürliche Gesteinsmehle, wie Kaoline, Tonerden, Talkum, Kreide, Quarz, Attapulgit, Montmorillonit der Diatomeenerde und synthetische Gesteinsmehle, wie hochdisperse Kieselsäure, Aluminiumoxid und Silikate. Als feste Trägerstoffe für Granulate kommen in Frage ; z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel. Als Emulgier- und/oder schaumerzeugende Mittel kommen in Frage : z.B. nichtionogene und anionische Emulgatoren, wie Polyoxyethylen-Fettsäureester, Polyoxyethylen-Fettalkoholether, z.B. Alkylarylpolyglycol-ether, Alkylsulfonate, Alkylsulfate, Arylsulfonate sowie Eiweißhydrolysate. Als Dispergiermittel kommen in Frage : z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe, wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in den Formulierungen in Mischung mit anderen bekannten Wirkstoffen vorliegen, wie Fungizide, Insektizide, Akarizide und Herbizide, sowie in Mischungen mit Düngemitteln und Wachstumsregulatoren.

Die Wirkstoffe können als solche, in Form ihrer Formulierungen oder den daraus bereiteten Anwendungsformen, wie gebrauchsfertige Lösungen, emulgierbare Konzentrate, Emulsionen, Schäume, Suspensionen, Spritzpulver, Pasten, lösliche Pulver, Stäubemittel und Granulate, angewendet werden. Die Anwendung geschieht in üblicher weise, z.B. durch Gießen, Verspritzen, Versprühen, Verstreuen, Verstäuben, Verschäumen, Bestreichen usw. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden. Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.-%, vorzugsweise zwischen 0,5 und 0,001 %.

Bei der Saatgutbehandlung werden im allgemeinen Wirkstoffmengen von 0,001 bis 50 g je Kilogramm Saatgut, vorzugsweise 0,01 bis 10 g, benötigt.

Bei Behandlung des Bodens sind Wirkstoffkonzentrationen von 0,00001 bis 0,1 Gew.-%, vorzugsweise von 0,0001 bis 0,02 %, am Wirkungsort erforderlich.

## Herstellungsbeispiele

### Beispiel 1

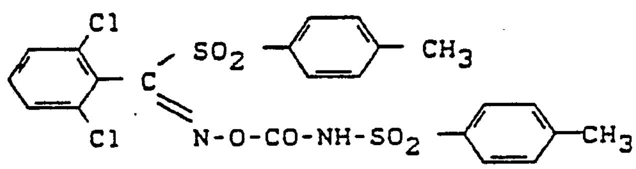

36 g (0,1 m) α-(4-Methyl-phenyl-sulfonyl)-2,6-dichlorbenzaldoxim werden in 200 ml Methylenchlorid gelöst und mit 19,7 g (0,1 m) 4-Methyl-phenyl-sulfonylisocyanat versetzt, wobei zur Vervollständigung der Reaktion einige Tropfen Triethylamin zugesetzt werden. Die Reaktion verläuft exotherm. Es wird über Nacht bei Raumtemperatur gerührt. Das Reaktionsprodukt fällt aus, wird abgesaugt, gewaschen und getrocknet. Man erhält 23 g (41 % der Theorie) der gewünschten Substanz mit einem Schmelzpunkt von 157 °C.

In analoger Weise wie Beispiel 1 und 31 werden die folgenden Verbindungen der Formel (I)

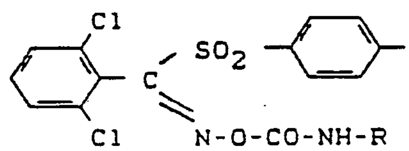

(I)

hergestellt :

| Beispiel Nr. | R | physikalische Daten (Schmelzpunkt °C) |
|---|---|---|
| 2 | $-C_2H_5$ | 182 |
| 3 | $-CH_3$ | 179 |
| 4 | 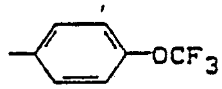 | 176 |

(Suite)

| Beispiel Nr. | R | physikalische Daten (Schmelzpunkt °C) |
|---|---|---|
| 5 | 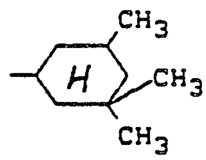 | 173 |
| 6 | $-C_3H_7$-iso | 187 |
| 7 | $-C_3H_7$-n | 184 |
| 8 | $-C_4H_9$-n | 177 |
| 9 | $-(CH_2)_6-Cl$ | 109 |
| 10 | $-C_4H_9$-iso | 192 |
| 11 | $-(CH_2)_5-CN$ | 168 |
| 12 | 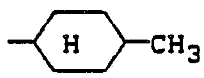 | 171 |
| 13 | 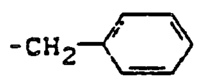 | 207 |
| 14 | 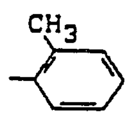 | 190 |
| 15 | 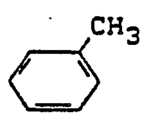 | 201 |
| 16 | 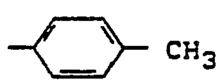 | 208 (Zers.) |
| 17 | 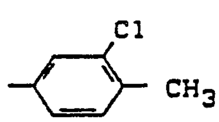 | 210 (Zers.) |
| 18 | 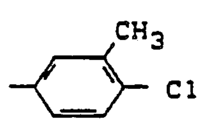 | 202 (Zers.) |
| 19 | 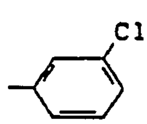 | 198 (Zers.) |

(Suite)

| Beispiel Nr. | R | physikalische Daten (Schmelzpunkt $^{0}C$) |
|---|---|---|
| 20 | 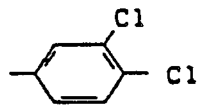 | 194 (Zers.) |
| 21 | 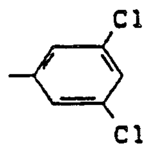 | 193 (Zers.) |
| 22 | 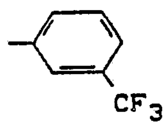 | 193 (Zers.) |
| 23 | 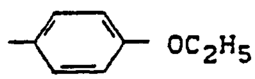 | 192 (Zers.) |
| 24 | 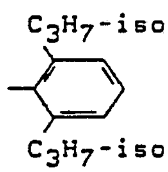 | 190 |
| 25 | $-C_4H_9$-tert. | 174 |
| 26 | 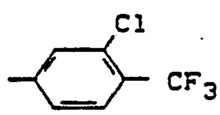 | 186 |
| 27 | 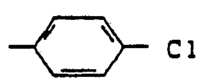 | 206 |
| 28 | 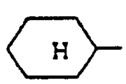 | 172 |
| 29 | 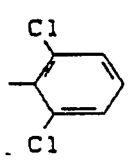 | 191 |
| 30 | $-C_4H_9$-iso | 192 |

Beispiel 31

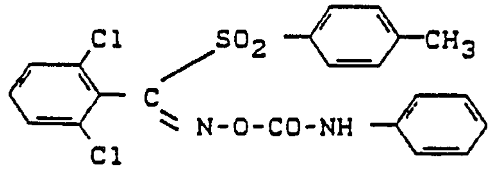

25,5 g (0,075 m) α-(4-Methyl-phenyl-sulfonyl)-2,6-dichlorbenzaldoxim wurden in 200 ml Methylenchlorid gelöst und mit 8,9 g (0,075 m) Phenylisocyanat in Gegenwart einiger Tropfen Triäthylamin versetzt. Die Reaktion ist exotherm. Das Reaktionsgemisch wird eine klare Lösung. Man läßt es über Nacht bei Raumtemperatur rühren, das Reaktionsprodukt ist dann ausgefallen. Das Reaktionsgemisch wird im Vakuum eingeengt, anschließend mit Aceton heiß verrieben, abgesaugt, gewaschen und getrocknet. Man erhält 10 g (29 % der Theorie) der gewünschten Substanz mit einem Schmelzpunkt von 198 °C.

Anwendungsbeispiele

In den nachfolgenden Anwendungsbeispielen werden die nachstehend aufgeführten Verbindungen als Vergleichssubstanzen verwendet :

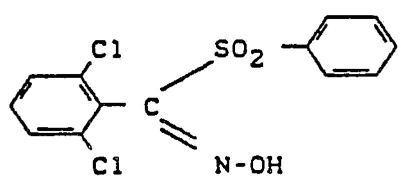

(A)

α-Phenylsulfonyl-2,6-dichlorbenzaldoxim und

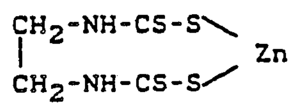

(B)

Zinkethylen-1,2-bis-dithiocarbamat

Beispiel A

Phytophthora-Test (Tomate)/protektiv

Lösungsmittel : 4,7 Gewichtsteile Aceton
Emulgator : 0,3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen mit einer wäßrigen Sporensuspension von Phytophthora infestans inokuliert.

Die Pflanzen werden in einer Inkubationskabine mit 100 % relativer Luftfeuchtigkeit und ca. 20 °C aufgestellt.

3 Tage nach der Inokulation erfolgt die Auswertung.

Eine deutliche Überlegenheit in der Wirksamkeit gegenüber dem Stand der Technik zeigen in diesem Test z.B. die Verbindungen gemäß den Herstellungsbeispielen : 1, 2, 3 und 4.

Beispiel B

Pyricularia-Test (Reis)/protektiv

Lösungsmittel : 12,5 Gewichtsteile Aceton
Emulgator : 0,3 Gewichtsteile Alkylarylpolyglykolether

Zur Herstellung einer zweckmäßigen Wirkstoffzubereitung vermischt man 1 Gewichtsteil Wirkstoff

mit der angegebenen Menge Lösungsmittel und verdünnt das Konzentrat mit Wasser und der angegebenen Menge Emulgator auf die gewünschte Konzentration.

Zur Prüfung auf protektive Wirksamkeit bespritzt man junge Reispflanzen mit der Wirkstoffzubereitung bis zur Tropfnässe. Nach dem Abtrocknen des Spritzbelages werden die Pflanzen mit einer wäßrigen Sporensuspension von Pyricularia oryzae inokuliert. Anschließend werden die Pflanzen in einem Gewächshaus bei 100 % rel. Luftfeuchtigkeit und 25 °C aufgestellt.

4 Tage nach der Inokulation erfolgt die Auswertung des Krankheitsbefalls.

Eine deutliche Überlegenheit in der Wirksamkeit gegenüber dem Stand der Technik zeigen in diesem Test z.B. die Verbindungen gemäß den Herstellungsbeispielen : 1, 3 und 2.

Beispiel C

Leptosphaeria nodorum-Test (Weizen)/protektiv

Lösungsmittel : 100 Gewichtsteile Dimethylformamid
Emulgator : 0,25 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit einer Konidiensuspension von Leptosphaeria nodorum besprüht. Die Pflanzen verbleiben 48 Stunden bei 20 °C und 100 % relativer Luftfeuchtigkeit in einer Inkubationskabine.

Die Pflanzen werden in einem Gewächshaus bei einer Temperatur von ca. 15 °C und einer relativen Luftfeuchtigkeit von ca. 80 % aufgestellt.

10 Tage nach der Inokulation erfolgt die Auswertung.

Eine deutliche Überlegenheit in der Wirksamkeit gegenüber dem Stand der Technik zeigt bei diesem Test z.B. die Verbindung gemäß dem Herstellungsbeispiel :

**Patentansprüche**

1. Benzaldoxim-carbamat-Derivate der Formel (I)

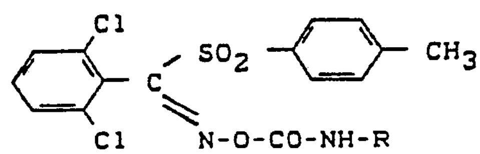

(I)

in welcher R für Aklyl mit 1 bis 6 Kohlenstoffatomen, für Halogenalkyl mit 1 bis 8 Kohlenstoffatomen und 1 bis 5 Halogenatomen, für Cyanalkyl mit 1 bis 6 Kohlenstoffatomen im Alkylteil, für Tosyl, für Benzyl, für gegebenenfalls ein- bis fünffach durch Alkyl mit 1 bis 4 Kohlenstoffatomen substituiertes Cycloalkyl mit 5 bis 7 Kohlenstoffatomen oder für gegebenenfalls ein- bis fünffach, gleich oder verschieden durch Alkyl mit 1 bis 6 Kohlenstoffatomen, Alkoxy mit 1 bis 4 Kohlenstoffatomen, Halogenalkoxy oder Halogenalkyl mit je 1 bis 4 Kohlenstoffatomen und 1 bis 5 Halogenatomen und Halogen substituiertes Phenyl steht.

2. Benzaldoxim-carbamat-Derivate der Formel (I) gemäß Anspruch 1, in welcher R für geradkettiges oder verzweigtes Alkyl mit 1 bis 4 Kohlenstoffatomen, für Halogenalkyl mit 1 bis 6 Kohlenstoffatomen und 1 bis 3 gleichen oder verschiedenen Halogenatomen, für Cyanalkyl mit 1 bis 5 Kohlenstoffatomen im Alkylteil, für Tosyl, für Benzyl, für gegebenenfalls ein- bis dreifach, gleich oder verschieden durch Alkyl mit 1 bis 3 Kohlenstoffatomen substituiertes Cycloalkyl mit 6 oder 7 Kohlenstoffatomen, für gegebenenfalls ein- bis dreifach, gleich oder verschieden durch Alkyl mit 1 bis 4 Kohlenstoffatomen, durch Alkoxy mit 1 bis 3 Kohlenstoffatomen, durch Halogenalkyl oder Halogenalkoxy mit je 1 bis 3 Kohlenstoffatomen und 1 bis 3 Halogenatomen und Halogen substituiertes Phenyl steht.

3. Benzaldoxim-carbamat-Derivate der Formel (I) gemäß Anspruch 1, in welcher R für Methyl, Ethyl, n-Propyl, iso-Propyl, n-Butyl, iso-Butyl, tert.-Butyl, 6-Chlor-n-hexyl, 5-Cyano-n-pentyl, Tosyl, Benzyl, für Cyclohexyl oder ein- bis dreifach durch Methyl oder Ethyl substituiertes Cyclohexyl, für Phenyl oder ein- bis dreifach, gleich oder verschieden durch Methyl, Ethyl, n-Propyl, iso-Propyl, Methoxy, Ethoxy, Trifluormethoxy, Trifluormethyl, Chlor und Fluor substituiertes Phenyl steht.

4. Benzaldoxim-carbamat-Derivate der Formel (I) gemäß Anspruch 1, in welcher R für Methyl, Ethyl, n-Propyl, iso-Propyl, n-Butyl, iso-Butyl, tert.-Butyl, 6-Chlor-n-hexyl, 5-Cyano-n-pentyl, Benzyl, Tosyl, Cyclohexyl, 3,5,5-Trimethyl-cyclohexyl, 4-Methyl-cyclohexyl, Phenyl, 4-Trifluormethoxy-phenyl, 2-, 3- oder 4-Methyl-phenyl, 3-Chlor-4-methyl-phenyl, 3-Methyl-4-chlorphenyl, 3- oder 4-Chlorphenyl, 2,6-, 3,4- oder

3,5-Dichlor-phenyl, 3-Trifluormethyl-phenyl, 4-Ethoxy-phenyl, 3-Chlor-4-trifluormethylphenyl oder 3,6-Di-isopropyl-phenyl steht.

5. Benzaldoxim-carbamat-Derivate der Formel (I) gemäß Anspruch 1, in welcher R für Methyl, Ethyl, Tosyl, 4-Trifluormethoxyphenyl oder 3,5,5-Trimethyl-cyclohexyl steht.

6. Verfahren zur Herstellung von Benzaldoxim-carbamat-Derivaten der Formel (I)

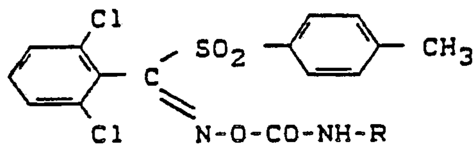

(I)

in welcher R für Alkyl mit 1 bis 6 Kohlenstoffatomen, für Halogenalkyl mit 1 bis 8 Kohlenstoffatomen und 1 bis 5 Halogenatomen, für Cyanalkyl mit 1 bis 6 Kohlenstoffatomen im Alkylteil, für Tosyl, für Benzyl, für gegebenenfalls ein- bis fünffach durch Alkyl mit 1 bis 4 Kohlenstoffatomen substituiertes Cycloalkyl mit 5 bis 7 Kohlenstoffatomen oder für gegebenenfalls ein- bis fünffach, gleich oder verschieden durch Alkyl mit 1 bis 6 Kohlenstoffatomen, Alkoxy mit 1 bis 4 Kohlenstoffatomen, Halogenalkoxy oder Halogenalkyl mit je 1 bis 4 Kohlenstoffatomen und 1 bis 5 Halogenatomen und Halogen substituiertes Phenyl steht, dadurch gekennzeichnet, daß man α-(4-Methyl-phenylsulfonyl)-2,6-dichlorbenzaldoxim der Formel (II)

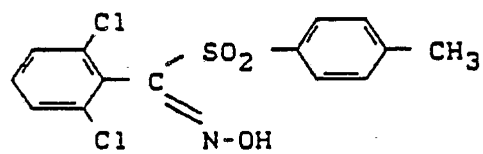

(II)

mit Isocyanaten der Formel (III)

$$R{-}NCO \qquad (III)$$

in welcher R die oben angegebene Bedeutung hat, gegebenenfalls in Gegenwart von Lösungs- oder Verdünnungsmitteln bei Temperaturen von 0 °C bis 100 °C umsetzt.

7. Schädlingsbekämpfungsmittel, gekennzeichnet durch einen Gehalt an mindestens einem Benzaldoxim-carbamat-Derivat der Formel (I) gemäß den Ansprüchen 1 und 6.

8. Verfahren zur Bekämpfung von Schädlingen, dadurch gekennzeichnet, daß man Benzaldoxim-carbamat-Derivate der Formel (I) gemäß den Ansprüchen 1 und 6 auf Schädlinge und/oder ihren Lebensraum einwirken läßt.

9. Verwendung von Benzaldoxim-carbamat-Derivaten der Formel (I) gemäß den Ansprüchen 1 und 6 zur Bekämpfung von Schädlingen.

10. Verfahren zur Herstellung von Schädlingsbekämpfungsmitteln, dadurch gekennzeichnet, daß man Benzaldoxim-carbamat-Derivate der Formel (I) gemäß den Ansprüchen 1 bis 6 mit Streckmitteln und/oder oberflächenaktiven Mitteln vermischt.

## Claims

1. Benzaldoxime carbamate derivatives of the formula (I)

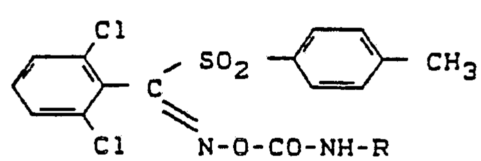

(I)

in which R represents alkyl having 1 to 6 carbon atoms, halogenoalkyl having 1 to 8 carbon atoms and 1 to 5 halogen atoms, cyanoalkyl having 1 to 6 carbon atoms in the alkyl part, tosyl, benzyl, cycloalkyl which has 5 to 7 carbon atoms and is optionally monosubstituted to pentasubstituted by alkyl having 1 to 4 carbon atoms, or phenyl which is optionally monosubstituted to pentasubstituted by identical or different substituents from amongst alkyl having 1 to 6 carbon atoms, alkoxy having 1 to 4 carbon atoms, halogenoalkoxy or halogenoalkyl having 1 to 4 carbon atoms and 1 to 5 halogen atoms and halogen.

2. Benzaldoxime carbamate derivatives of the formula (I) according to Claim 1, in which R represents straight-chain or branched alkyl having 1 to 4 carbon atoms, halogenoalkyl having 1 to 6 carbon atoms and 1 to 3 identical or different halogen atoms, cyanoalkyl having 1 to 5 carbon atoms in the alkyl part, tosyl or benzyl, or represents cycloalkyl which has 6 or 7 carbon atoms and is optionally monosubstituted to trisubstituted by identical or different substituents from amongst alkyl having 1 to 3 carbon atoms, or

represents phenyl which is optionally monosubstituted to trisubstituted by identical or different substituents from amongst alkyl having having 1 to 4 carbon atoms, alkoxy having 1 to 3 carbon atoms, halogenoalkyl or halogenoalkoxy, each having 1 to 3 carbon atoms and 1 to 3 halogen atoms, and halogen.

3. Benzaldoxime carbamate derivatives of the formula (I) according to Claim 1, in which R represents methyl, ethyl, n-propyl, iso-propyl, n-butyl, iso-butyl, tert.-butyl, 6-chloro-n-hexyl, 5-cyano-n-pentyl, tosyl, benzyl, cyclohexyl or cyclohexyl which is monosubstituted to trisubstituted by methyl or ethyl ; phenyl or phenyl which is monosubstituted to tri-substituted by identical or different substituents from amongst methyl, ethyl, n-propyl, iso-propyl, methoxy, ethoxy, trifluoromethoxy, trifluoromethyl, chlorine and fluorine.

4. Benzaldoxime carbamate derivatives of the formula (I) according to Claim 1, in which R represents methyl, ethyl, n-propyl, iso-propyl, n-butyl, iso-butyl, tert.-butyl, 6-chloro-n-hexyl, 5-cyano-n-pentyl, benzyl, tosyl, cyclohexyl, 3,5,5-trimethyl-cyclohexyl, 4-methyl-cyclohexyl, phenyl, 4-trifluoromethoxyphenyl, 2-, 3- or 4-methyl-phenyl, 3-chloro-4-methyl-phenyl, 3-methyl-4-chlorophenyl, 3- or 4-chlorophenyl, 2,6-, 3,4- or 3,5-dichloro-phenyl, 3-trifluoromethyl-phenyl, 4-ethoxy-phenyl, 3-chloro-4-trifluoromethylphenyl or 3,6-di-isopropyl-phenyl.

5. Benzaldoxime carbamate derivatives of the formula (I) according to Claim 1 in which R represents methyl, ethyl, tosyl, 4-trifluoro-methoxyphenyl or 3,5,5-trimethyl-cyclohexyl.

6. Process for the preparation of benzaldoxime carbamate derivatives of the formula (I)

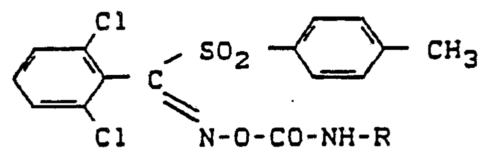

(I)

in which R represents alkyl having 1 to 6 carbon atoms, halogenoalkyl having 1 to 8 carbon atoms and 1 to 5 halogen atoms, cyanoalkyl having 1 to 6 carbon atoms in the alkyl part, tosyl, benzyl, cycloalkyl which has 5 to 7 carbon atoms and is optionally monosubstituted to pentasubstituted by alkyl having 1 to 4 carbon atoms, or phenyl which is optionally monosubstituted to pentasubstituted by identical or different substituents from amongst alkyl having 1 to 6 carbon atoms, alkoxy having 1 to 4 carbon atoms, halogenoalkoxy or halogenoalkyl having 1 to 4 carbon atoms and 1 to 5 halogen atoms and halogen, characterized in that α-(4-methyl-phenylsulphonyl)-2,6-dichlorobenzaldoxime of the formula (II)

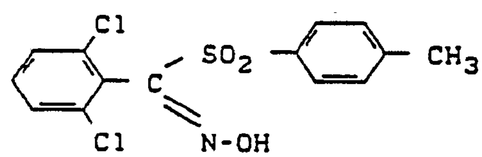

(II)

is reacted with isocyanates of the formula (III)

R—NCO (III)

in which R has the meaning given above, if appropriate in the presence of solvents or diluents at temperatures from 0 °C to 100 °C.

7. Pest-combating agents, characterized in that they contain at least one benzaldoxime carbamate derivative of the formula (I) according to Claims 1 and 6.

8. Method of combating pests, characterized in that benzaldoxime carbamate derivatives of the formula (I) according to Claims 1 and 6 are allowed to act on pests and/or their habitat.

9. Use of benzaldoxime carbamate derivatives of the formula (I) according to Claims 1 and 6 for combating pests.

10. Process for the preparation of pest-combating agents, characterized in that benzaldoxime carbamate derivatives of the formula (I) according to Claims 1 to 6 are mixed with extenders and/or surface-active agents.

**Revendications**

1. Dérivés de carbamate de benzaldoxime de formule (I)

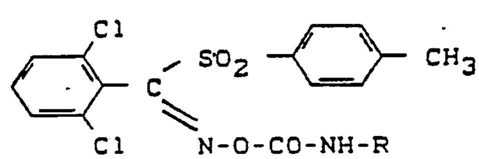

(I)

dans laquelle R représente un alcoyle ayant 1 à 6 atomes de carbone, un halogénoalcoyle ayant 1 à 8 atomes de carbone et 1 à 5 atomes d'halogène, un cyanoalcoyle ayant 1 à 6 atomes de carbone dans la portion alcoyle, un tosyle, un benzyle, un cycloalcoyle ayant 5 à 7 atomes de carbone éventuellement substitué une à cinq fois par un alcoyle ayant 1 à 4 atomes de carbone, ou un phényle éventuellement substitué une à cinq fois, de manière identique ou différente, par un alcoyle ayant 1 à 6 atomes de carbone, un alkoxy ayant 1 à 4 atomes de carbone, un halogénoalcoxy ou un halogénoalcoyle ayant chacun 1 à 4 atomes de carbone et 1 à 5 atomes d'halogène, et par de l'halogène.

2. Dérivés de carbamate de benzaldoxime de formule (I) selon la revendication 1, dans laquelle R représente un alcoyle à chaîne droite ou ramifiée ayant 1 à 4 atomes de carbone, un halogénoalcoyle ayant 1 à 6 atomes de carbone et 1 à 3 atomes d'halogène, identiques ou différents, un cyanoalcoyle ayant 1 à 5 atomes de carbone dans la portion alcoyle, un tosyle, un benzyle, un cycloalcoyle ayant 6 ou 7 atomes de carbone éventuellement substitué une à trois fois, de manière identique ou différente, par un alcoyle ayant 1 à 3 atomes de carbone, un phényle éventuellement substitué une à trois fois, de manière identique ou différente, par un alcoyle ayant 1 à 4 atomes de carbone, par un alcoxy ayant 1 à 3 atomes de carbone, par un halogénoalcoyle ou un halogénoalcoxy ayant chacun 1 à 3 atomes de carbone et 1 à 3 atomes d'halogène, et par de l'halogène.

3. Dérivés de carbamate de benzaldoxime de formule (I) selon la revendication 1, dans laquelle R représente un méthyle, éthyle, n-propyle, isopropyle, n-butyle, isobutyle, t-butyle, 6-chloro-n-hexyle, 5-cyano-n-pentyle, tosyle, benzyle, un cyclohexyle ou un cyclohexyle substitué une à trois fois par un méthyle ou éthyle, un phényle ou un phényle substitué une à trois fois, de manière identique ou différente, par un méthyle, éthyle, n-propyle, isopropyle, méthoxy, éthoxy, trifluorométhoxy, trifluorométhyle, du chlore et du fluor.

4. Dérivés de carbamate de benzaldoxime de formule (I) selon la revendication 1, dans laquelle R représente un méthyle, éthyle, n-propyle, isopropyle, n-butyle, isobutyle, t-butyle, 6-chloro-n-hexyle, 5-cyano-n-pentyle, benzyle, tosyle, cyclohexyle, 3,5,5-triméthyl-cyclohexyle, 4-méthyl-cyclohexyle, phényle, 4-trifluorométhoxy-phényle, 2-, 3- ou 4-méthyl-phényle, 3-chloro-4-méthyl-phényle, 3-méthyl-4-chlorophényle, 3- ou 4-chlorophényle, 2,6-, 3,4- ou 3,5-dichloro-phényle, 3-trifluorométhyl-phényle, 4-éthoxy-phényle, 3-chloro-4-trifluorométhyl-phényle ou 3,6-di-isopropyl-phényle.

5. Dérivés de carbamate de benzaldoxime de formule (I) selon la revendication 1, dans laquelle R représente un méthyle, éthyle, tosyle, 4-trifluorométhoxyphényle ou 3,5,5-triméthyl-cyclohexyle.

6. Procédé de préparation de dérivés de carbamate de benzaldoxime de formule (I)

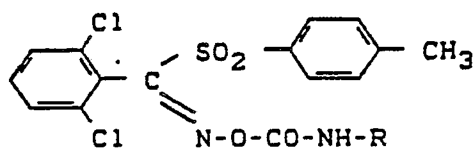

(I)

dans laquelle R représente un alcoyle ayant 1 à 6 atomes de carbone, un halogénoalcoyle ayant 1 à 8 atomes de carbone et 1 à 5 atomes d'halogène, un cyanoalcoyle ayant 1 à 6 atomes de carbone dans la portion alcoyle, un tosyle, un benzyle, un cycloalcoyle ayant 5 à 7 atomes de carbone éventuellement substitué une à cinq fois par un alcoyle ayant 1 à 4 atomes de carbone, ou un phényle éventuellement substitué une à cinq fois de manière identique ou différente, par un alcoyle ayant 1 à 6 atomes de carbone, un alcoxy ayant 1 à 4 atomes de carbone, un halogénoalcoxy ou un halogénoalcoyle ayant chacun 1 à 4 atomes de carbone et 1 à 5 atomes d'halogène, et par de l'halogène, caractérisé en ce qu'on fait réagir de l'α-(4-méthyl-phényl-sulfonyl)-2,6-dichlorobenzaldoxime de formule (II)

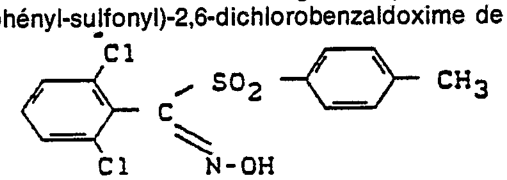

(II)

avec des isocyanates de formule (III)

R—NCO (III)

dans laquelle R a la signification indiquée, éventuellement en présence de solvants ou de diluants à des températures de 0 °C à 100 °C.

7. Agents antiparasitaires, caractérisés par une teneur en au moins un dérivé de carbamate de benzaldoxime de formule (I) selon les revendications 1 et 6.

8. Procédé pour combattre les parasites, caractérisé en ce qu'on fait agir des dérivés de carbamate de benzaldoxime de formule (I) selon les revendications 1 et 6 sur les parasites et/ou sur leur espace vital.

9. Utilisation des dérivés de carbamate de benzaldoxime de formule (I) selon les revendications 1 et 6 pour combattre les parasites.

10. Procédé de fabrication d'agents antiparasitaires, caractérisé en ce qu'on mélange les dérivés de carbamate de benzaldoxime de formule (I) selon les revendications 1 à 6 avec des diluants et/ou des agents tensioactifs.